# EUROPEAN PATENT APPLICATION

(11) **EP 4 353 350 A1**
(43) Date of publication of application: **17.04.2024**
(21) Application number: 22820334.5
(22) Date of filing: 10.06.2022
(51) Int. Cl.: B01J 13/14, B01J 2/02

(54) **CAPSULE PRODUCTION METHOD AND CAPSULE PRODUCTION DEVICE**

(30) Priority: 11.06.2021 JP 2021098149
(71) Applicant: Kagoshima University, Kagoshima-shi, Kagoshima 890-8580 (JP)
(72) Inventor: TAKEI Takayuki, Kagoshima-shi, Kagoshima 890-8580 (JP); YOSHIDA Masahiro, Kagoshima-shi, Kagoshima 890-8580 (JP); OHZUNO Yoshihiro, Kagoshima-shi, Kagoshima 890-8580 (JP)
(74) Representative: v. Bezold & Partner Patentanwälte - PartG mbB
(86) International application number: PCT/JP2022/023403
(87) International publication number: WO 2022/260156

(57) **Abstract**

A tubular body (111) has a shape of tube surrounding a virtual center line (VL). A guide groove (112) that is helical about the virtual center line (VL) is provided in the inner surface of the tubular body (111). A rotation device (120) rotates the tubular body (111) around the virtual center line (VL). A dropping device (130) drops, on the inner surface of the tubular body (111), an inclusion substance and a wall material precursor liquid that is a precursor of a wall material covering the inclusion substance. A liquid droplet dropped by the dropping device (130) rolls in the guide groove (112) of the tubular body (111), the tubular body (111) being rotated by the rotation device (120).

## Description

### Technical Field

The present disclosure relates to a capsule production method and a capsule production device.

### Background Art

As disclosed in Patent Literature 1, a capsule having a structure in which an inclusion substance is covered by a wall material is known. Inclusion substances are, for example, desired materials such as agricultural chemicals, inks, pharmaceutical products, and the like. The wall material functions in enhancing handleability of the inclusion substance, protecting the inclusion substance from the external environment, and controlling a release rate of the inclusion substance.

Patent Literature 1 proposes a capsule production method as described below. First, a liquid droplet of a raw material liquid containing a monomer liquid or a polymer liquid that is a precursor of the wall material, and the inclusion substance is dropped onto the surface of a flat plate. The dropped liquid droplet maintains the spherical shape by surface tension. Next, the monomer liquid or the polymer liquid is solidified so as to be the wall material. Consequently, a capsule is obtained.

### Citation List

### Patent Literature

Patent Literature 1: Unexamined Japanese Patent Application Publication No. 2016-087479

### Summary of Invention

### Technical Problem

According to the above-described conventional capsule production method, when a large number of liquid droplets are placed on the flat plate, unification, two liquid droplets or three or more liquid droplets being unified into a singular droplet, is likely to occur. This is one of the factors that make an efficient capsule production difficult.

An objective of the present disclosure is to provide a capsule production method capable of efficiently producing a capsule, and a capsule production device applicable in the implementation of such a capsule production method.

### Solution to Problem

A capsule production method according to the present disclosure to be performed by utilizing a tubular body having a shape of tube surrounding a virtual center line, the tubular body including a guide groove that is helical or circles about the virtual center line, the guide groove being provided in an inner surface of the tubular body, includes:
a dropping step of dropping, on the inner surface of the tubular body, an inclusion substance and a wall material precursor liquid that is a precursor of a wall material covering the inclusion substance;
an encapsulating step of progressing an encapsulation of the inclusion substance and the wall material precursor liquid by a liquid droplet dropped in the dropping step rolling in the guide groove of the tubular body, the tubular body rotating around the virtual center line; and
a capsule ejecting step of ejecting, from the tubular body, a capsule obtained by a progress of the encapsulation.

The above-described method may further include a powder placing step, prior to the dropping step, of placing powders of solid particulates in the guide groove of the tubular body in advance, the solid particulates being each smaller than the liquid droplet, wherein
in the encapsulating step, a liquid marble may be formed by the liquid droplet rolling together with the solid particulates in the guide groove of the tubular body rotating, and the liquid droplet may roll in the guide groove in a form of the liquid marble, the liquid marble being the liquid droplet having a surface that is dusted with the solid particulates.

In the above-described method, a surface of the guide groove of the tubular body may be liquid-repellent to the liquid droplet.

In the above-described method, an amount of the liquid droplet to be dropped at a time in the dropping step may be equal to or less than 15 µL, and a circumferential velocity of the inner surface of the tubular body in the dropping step and in the encapsulating step may be equal to or faster than 0.08 m/s and equal to or slower than 0.24 m/s.

In the above-described method, the wall material precursor liquid may have a characteristic of gelation, and the encapsulation may progress by the gelation of the wall material precursor liquid.

In the above-described method, the wall material precursor liquid may contain a monomer, and the encapsulation may progress by polymerization of the monomer.

In the above-described method, the wall material precursor liquid may contain a solvent, and the encapsulation may progress by elimination of the solvent.

A capsule production device according to the present disclosure includes:
a tubular body having a shape of tube surrounding a virtual center line, the tubular body including a guide groove that is helical or circles about the virtual center line, the guide groove being provided in an inner surface of the tubular body;
a rotation device to cause the tubular body to rotate around the virtual center line; and
a dropping device to drop, on the inner surface of the tubular body, an inclusion substance and a wall material precursor liquid that is a precursor of a wall material covering the inclusion substance, wherein
an encapsulation of the inclusion substance and the wall material precursor liquid progresses by a liquid droplet dropped by the dropping device rolling in the guide groove of the tubular body being rotated by the rotation device.

In the above-described device, an amount of the liquid droplet to be dropped at a time by the dropping device may be equal to or less than 15 µL.

The above-described device may further include a solidification facilitating device to facilitate solidification of the wall material precursor liquid in the liquid droplet that is rolling in the guide groove of the tubular body.

### Advantageous Effects of Invention

The capsule production method of the present disclosure achieves an efficient production of capsules.

### Brief Description of Drawings

FIG. 1A is a conceptual diagram illustrating an example capsule;
FIG. 1B is a conceptual diagram illustrating another example capsule;
FIG. 1C is a conceptual diagram illustrating still another example capsule;
FIG. 2 is a conceptual diagram illustrating a structure of a capsule production device according to Embodiment 1;
FIG. 3 is a conceptual diagram illustrating a major portion of a dropping device according to Embodiment 1;
FIG. 4 is a flowchart of a capsule production according to Embodiment 1;
FIG. 5 is a cross-sectional view illustrating a guide groove in a tubular body according to Embodiment 1 in an enlarged manner;

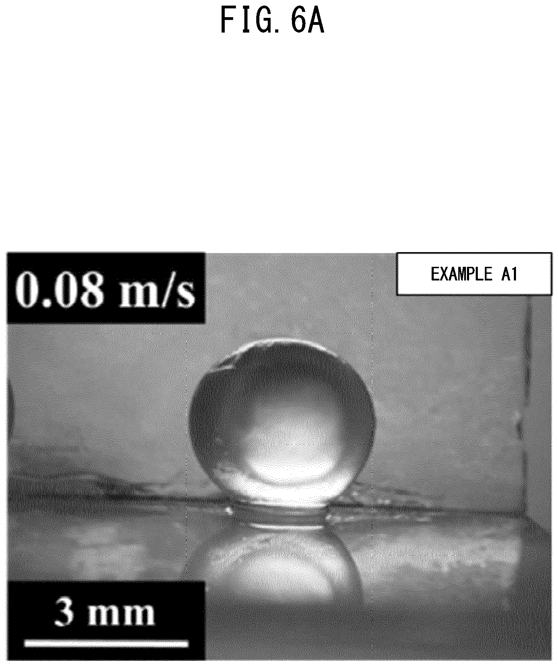

FIG. 6A is a micrograph of a capsule according to Example A1;

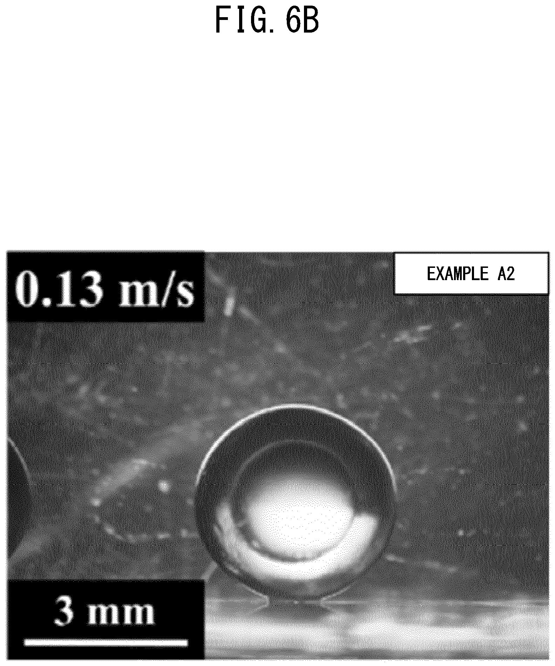

FIG. 6B is a micrograph of a capsule according to Example A2;

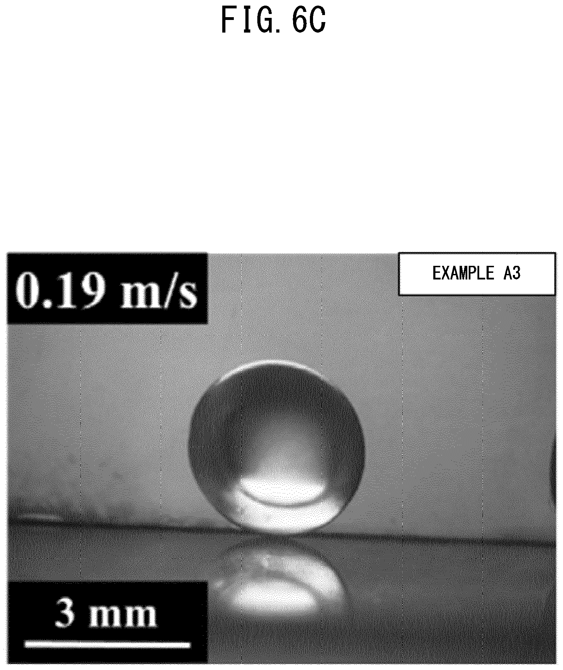

FIG. 6C is a micrograph of a capsule according to Example A3;

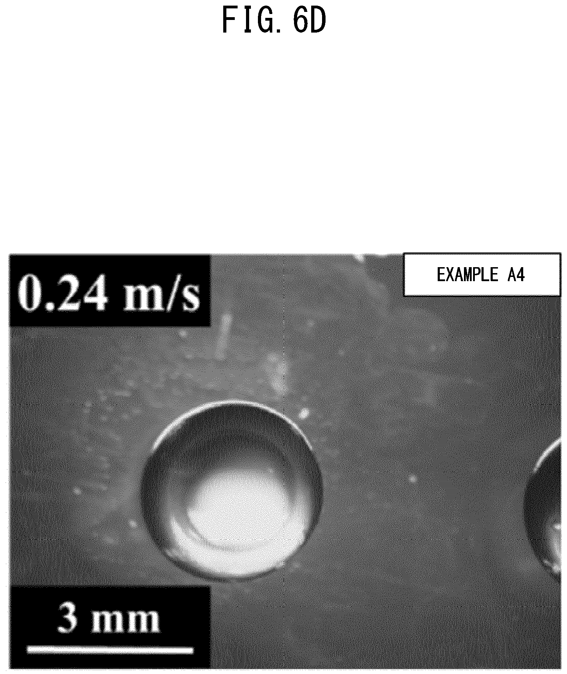

FIG. 6D is a micrograph of a capsule according to Example A4;

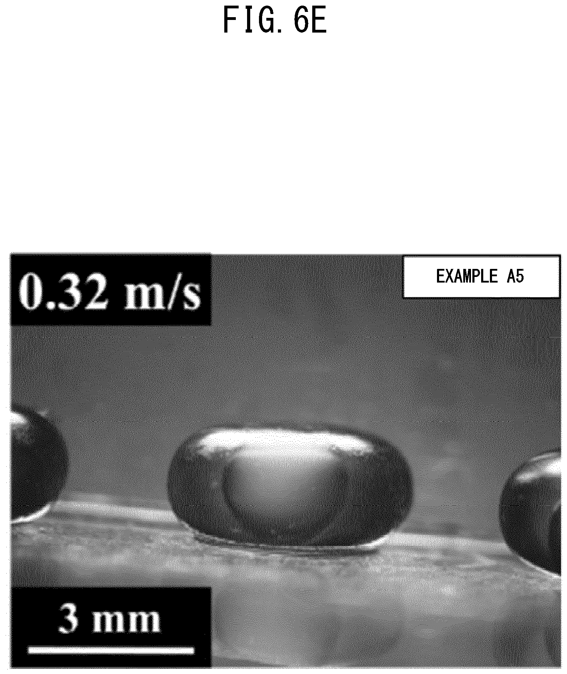

FIG. 6E is a micrograph of a capsule according to Example A5;

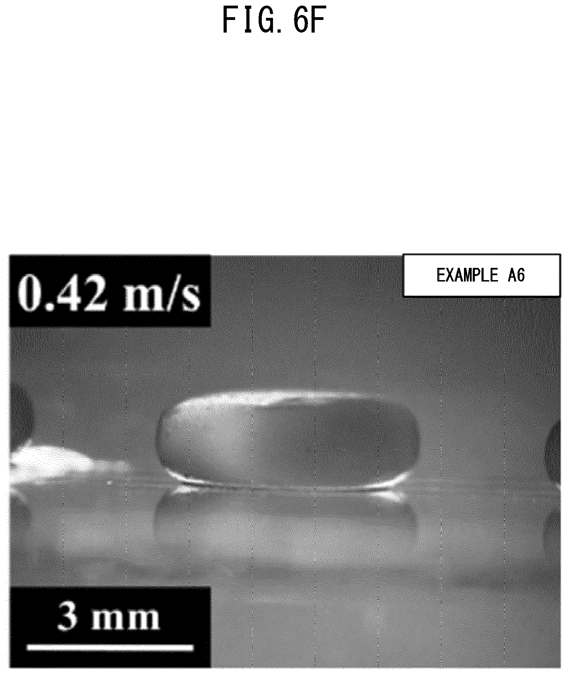

FIG. 6F is a micrograph of a capsule according to Example A6;
FIG. 7A is a micrograph of a capsule to describe a definition of sphericity;
FIG. 7B is a micrograph of a capsule to describe a definition of wall-thickness uniformity;
FIG. 8 is a graph showing the relation among, in the capsule production device, the circumferential velocity of the inner surface of a tubular body, the sphericity of an obtained capsule, the wall-thickness uniformity, and a success rate;
FIG. 9 is a micrograph of a capsule according to Example B;
FIG. 10 is a cross-sectional view of a guide groove in a tubular body according to Embodiment 2 in an enlarged manner;
FIG. 11 is a conceptual diagram illustrating a structure of a capsule production device according to Embodiment 3; and
FIG. 12 is a conceptual diagram illustrating a structure of a capsule production device according to Modified Example.

### Description of Embodiments

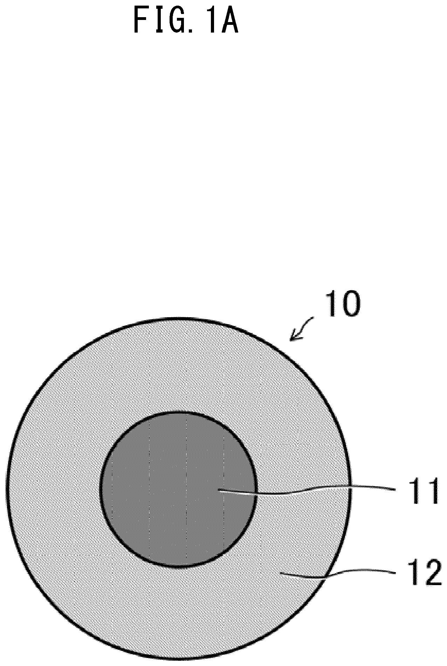

With reference to FIGS. 1A to 1C, first, example forms of a capsule are described.

FIG. 1A illustrates an example of a capsule 10. The capsule 10 has a structure in which an inclusion substance 11 is covered by a wall material 12. In this example, the inclusion substance 11 is present as a single, unitary and continuous region in the capsule 10. In the following description, such a form is referred to as "mononuclear".

Note that the inclusion substance 11 is, for example, a desired material such as agricultural chemical, ink, pharmaceutical product, or the like. The wall material 12 functions in enhancing handleability of the inclusion substance 11, protecting the inclusion substance 11 from the external environment, and controlling the release rate of the inclusion substance 11.

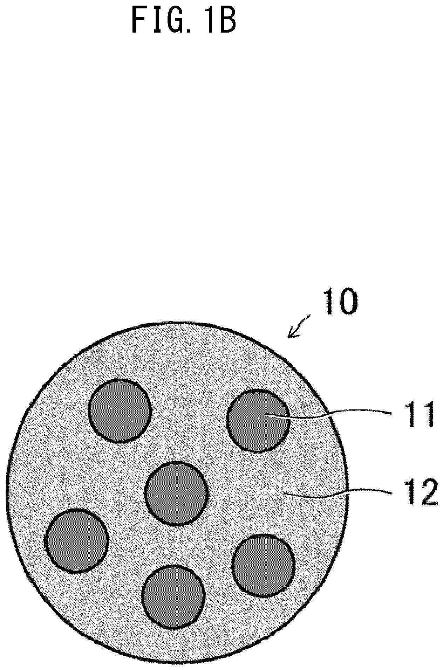

FIG. 1B illustrates another example of a capsule 10. In this example, the inclusion substances 11 are discretely distributed in multiple regions in the capsule 10. In the following description, such a form is referred to as "multinuclear".

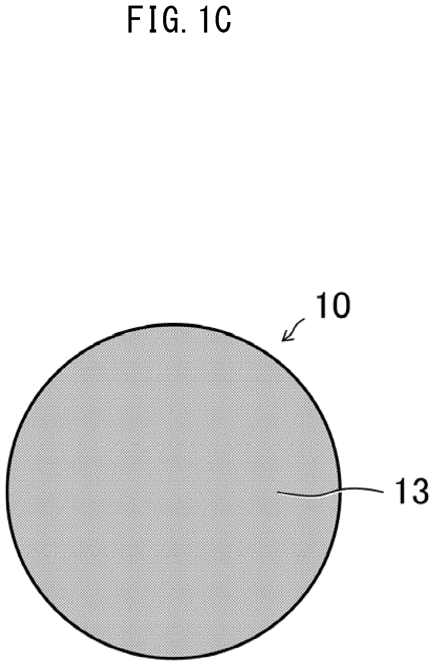

FIG. 1C illustrates still another example of a capsule 10. In this example, the interior of the capsule 10 is fully filled with a single kind of composite matrix 13 having a sequential composition. The composite matrix 13 includes the above-described inclusion substance 11 and wall material 12 mixed in a molecular level. Even if the capsule 10 according to this example is observed using an electron microscope, it is difficult to confirm a boundary between the wall material 12 and the inclusion substance 11.

Note that the structure illustrated in FIG. 1C is also referred to as "bead". In the present specification, however, the concept of a "capsule" also includes "bead". Moreover, a structure in which the mononuclear inclusion substance 11 illustrated in FIG. 1A or the multinuclear inclusion substance 11 illustrated in FIG. 1B is covered by the composite matrix 13 illustrated in FIG. 1C is also included in the concept of a "capsule". Furthermore, a structure in which the wall material 12 illustrated in FIG. 1A or FIG. 1B and the composite matrix 13 illustrated in FIG. 1C are co-existed is also included in the concept of a "capsule".

The typical diameter of the above-described capsule 10 is equal to or greater than 10 µm and is equal to or less than 1 cm, and more specifically, is equal to or greater than 100 µm and is equal to or less than 5 mm. The diameter of the capsule 10 can be measured by observation with a stereoscopic microscope or using a laser diffraction scattering method.

A capsule production device that produces the capsule 10 is described below.

### [Embodiment 1]

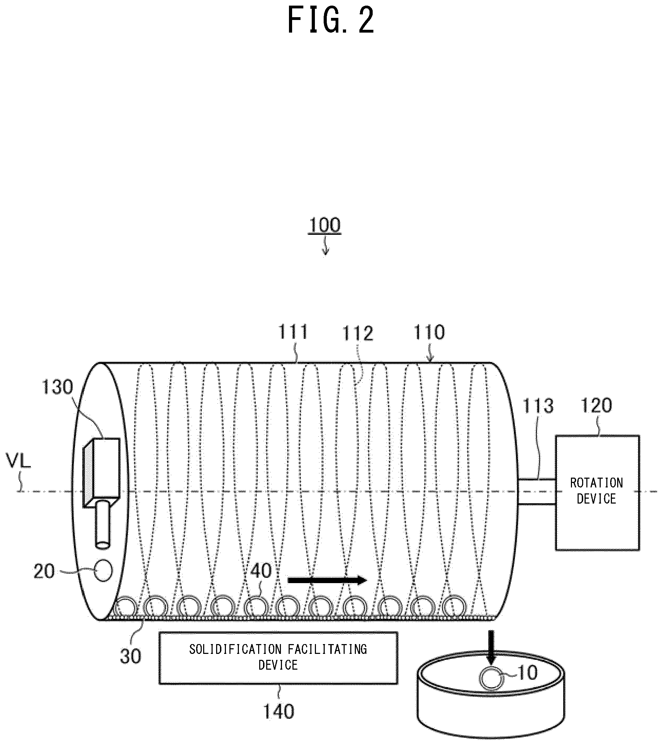

As illustrated in FIG. 2, a capsule production device 100 according to the present embodiment includes a rotating tube 110, a rotation device 120 that rotates the rotating tube 110, and a dropping device 130 that decreases, to the rotating tube 110 that has been rotated by the rotation device 120, a raw material liquid 20 that is a precursor of the capsule 10.

The rotating tube 110 includes a cylindrical body 111 having a shape of cylinder surrounding a virtual center line VL, and a rotation shaft 113 fastened to the cylindrical body 111.

The rotation shaft 113 is fastened to one end portion, of the cylindrical body 111, in the lengthwise direction parallel to the virtual center line VL. The rotation shaft 113 extends and is present on the virtual center line VL. Moreover, a guide groove 112 that helically extends around the virtual center line VL is provided in the inner surface of the cylindrical body 111. The guide groove 112 extends from the one end portion, of the cylindrical body 111, in the lengthwise direction to the other end portion thereof.

The rotating tube 110 is placed in an attitude falling sideways with the rotation shaft 113 being fallen down sideways. More specifically, in the present embodiment, the rotating tube 110 is fallen down horizontally, and the lengthwise direction of the cylindrical body 111 is consistent with the horizontal direction. The rotation device 120 rotates the cylindrical body 111 around the virtual center line VL through the rotation shaft 113.

The dropping device 130 is placed to the end portion of the cylindrical body 111 at, with respect to the lengthwise direction thereof, the opposite side to the end portion where the rotation shaft 113 is fastened.

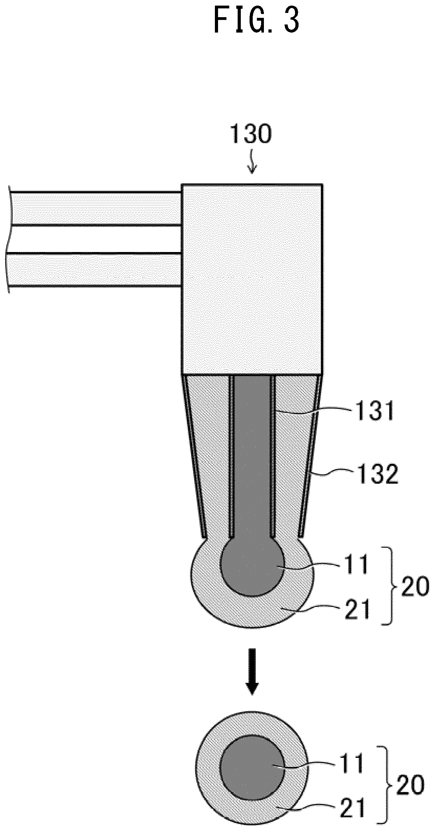

As illustrated in FIG. 3, the dropping device 130 includes an inner pipe 131, and an outer pipe 132 surrounding the outer side of the inner pipe 131 in a manner concentric with the inner pipe 131. A dual pipe includes the inner pipe 131 and the outer pipe 132. The inner pipe 131 discharges the inclusion substance 11 in liquid form. The outer pipe 132 discharges a wall material precursor liquid 21 that is a precursor of the wall material 12 illustrated in FIG. 1A.

The discharging of the inclusion substance 11 from the inner pipe 131 and the discharging of the wall material precursor liquid 21 from the outer pipe 132 are performed simultaneously. Hence, the liquid droplet of the raw material liquid 20 in a form in which the liquid inclusion substance 11 is covered by the wall material precursor liquid 21 is repeatedly dropped from the dropping device 130. Note that the diameter of the single droplet of the raw material liquid 20 is, for example, equal to or greater than 0.1 mm and is equal to or less than 5 mm.

The dropped liquid droplet of the raw material liquid 20 lands at the inner surface of the cylindrical body 111 illustrated in FIG. 2. It is preferable that the distance between the tip of the inner pipe 131 of the dropping device 130 and the tip of the outer pipe 132 thereof, and the inner surface of the cylindrical body 111 is preferably be equal to or less than 5 cm, more preferably be equal to or less than 2.5 cm, further preferably be equal to or less than 1.5 cm. The solidified liquid droplet of the raw material liquid 20 becomes the capsule 10 illustrated in FIG. 1A.

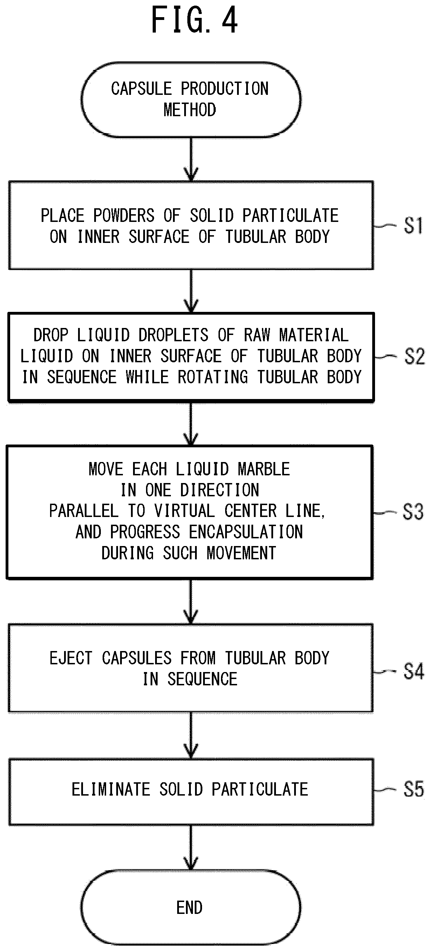

With reference to a flowchart illustrated in FIG. 4, a capsule production method according to the present embodiment is specifically described below.

First, as illustrated in FIG. 2, powders of the solid particulates 30 are placed on the inner surface of the cylindrical body 111 (a powder placing step S1). The powers of the solid particulates 30 in powder form are also placed in the guide groove 112.

Next, the liquid droplets of the raw material liquid 20 are dropped one after another by the dropping device 130 on the inner surface of the cylindrical body 111 while the cylindrical body 111 is being rotated by the rotation device 120 (a dropping step S2). Note that the amount of the liquid droplet to be dropped at a time by the dropping device 130 is, for example, equal to or less than 15 µL.

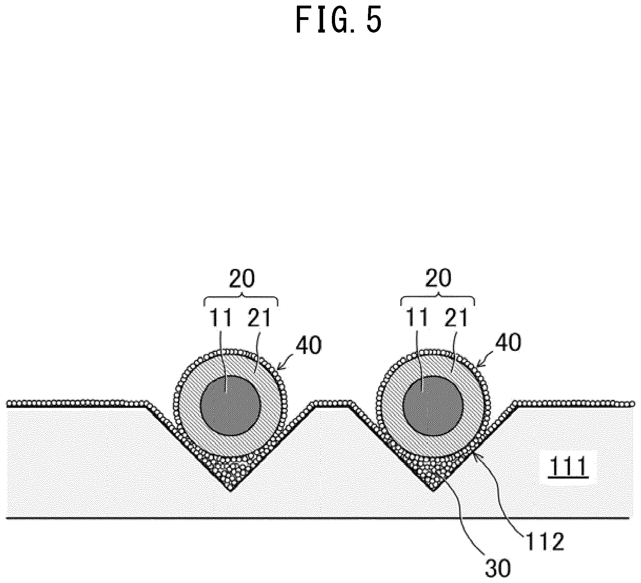

With reference to FIG. 5, the behavior of the liquid droplets dropped in the dropping step S2 is described. Each of the liquid droplets of the raw material liquid 20 rolls in the guide groove 112 in the inner surface of the rotating cylindrical body 111 together with the solid particulates 30. This forms a liquid marble 40 in which the surface of the liquid droplet of the raw material liquid 20 is dusted with the solid particulates 30.

The solid particulates 30 in the liquid marble 40 function as stabilizing the shape of the liquid droplet of the raw material liquid 20 into a spherical shape, and suppressing a unification of the multiple liquid droplets of the raw material liquid 20 into a singular droplet.

Each of the liquid droplets of the raw material liquid 20 rolls in the guide groove 112 in the form of the liquid marble 40 as described above. Since the guide groove 112 has a helical shape, each rolling liquid marble 40 slides in the circumferential direction of the cylindrical body 111, and moves in a direction parallel to the virtual center line VL illustrated in FIG. 2, more specifically, in a direction from the dropping device 130 toward the rotation shaft 113. Moreover, during such a movement, encapsulation progresses (an encapsulating step S3).

In the present embodiment, the term "encapsulation" means that the form of the inclusion substance 11 and that of the wall material precursor liquid 21 become approximate to the form of the capsule 10. More specifically, in the present embodiment, the concept of "encapsulation" includes the solidification of the wall material precursor liquid 21 in the liquid marble 40.

Subsequently, as the encapsulation of each liquid droplet of the raw material precursor liquid 20, more specifically, the solidification of the wall material precursor liquid 21 in each liquid marble 40 progresses, the liquid droplet of the raw material liquid 20 becomes the capsule 10, and such a capsule 10 is ejected in sequence from the cylindrical body 111 (a capsule ejecting step S4). For example, from the liquid droplet of the raw material liquid 20 that is equal to or greater than 1 µL and 10 µL, the capsule 10 with a diameter that is substantially equal to or greater than 1.0 mm and equal to or less than 3 mm is obtained.

However, the solid particulates 30 are adhered to the capsule 10 ejected from the cylindrical body 111. Hence, the solid particulates 30 adhered to the capsule 10 may be eliminated as needed (a solid particulate eliminating step S5) after the capsule ejecting step S4.

As discussed above, according to the present embodiment, each of the liquid droplets of the raw material liquid 20 moves within the cylindrical body 111 in one direction while rolling in the guide groove 112 in the form of the liquid marble 40. This suppresses the unification of the multiple liquid droplets of the raw material liquid 20 into a singular droplet, and enables an efficient production of the large number of the capsules 10.

Moreover, dusting of the liquid droplet of the raw material liquid 20 with the solid particulates 30 and the solidification of the wall material precursor liquid 21 can be performed successively. This also contributes to the efficient production of the large number of the capsules 10.

Furthermore, since the solidification of the wall material precursor liquid 21 progresses while the liquid marble 40 is rolling, the capsule 10 with a high sphericity can be obtained. The term sphericity is an evaluation index that represents the approximation of the shape of the capsule 10 to the shape of a true sphere.

Still further, since the solidification of the wall material precursor liquid 21 progresses while the liquid marble 40 is rolling, even when the mass density of the wall material precursor liquid 21 and that of the inclusion substance 11 differ, in particular, even when the mass density of the wall material precursor liquid 21 is greater than the density of the inclusion substance 11, the position of the inclusion substance 11 in the obtained capsule 10 is less likely to be displaced.

In particular, when the mononuclear capsule 10 illustrated in FIG. 1A is produced, the capsule 10 in which the wall material 12 has a unified thickness can be obtained. This also contributes to the improvement in the yield of the capsule 10.

The above-described solid particulate 30 is described more specifically in the following.

The solid particulate 30 has the diameter that is smaller than that of the liquid droplet of the raw material liquid 20 dropped from the dropping device 130. More specifically, the diameter of the solid particulate 30 is small to an extent that the coating of the liquid droplet of the raw material liquid 20 is possible. More specifically, the diameter of the solid particulate 30 is, for example, equal to or greater than 0.01 µm and equal to or less than 500 µm, preferably equal to or greater than 1 µm and equal to or less than 300 µm. Note that, in the present specification, the diameter of the solid particulate 30 refers to the average particle diameter with reference to the number of diameters measured by observation with a stereoscopic microscope or a laser diffraction scattering method.

The solid particulate 30 preferably has appropriate liquid-repellent characteristics against the wall material precursor liquid 21 in order to stably coat the liquid droplet of the raw material liquid 20. More specifically, it is preferable that the solid particulate 30 indicates, a contact angle, to the wall material precursor liquid 21, that is equal to or greater than 70 degrees, preferably equal to or greater than 100 degrees.

For example, the water-repellent solid particulate 30 that indicates a contact angle, to water, that is equal to or greater than 70 degrees, preferably equal to or greater than 100 degrees is applicable. Example water-repellent solid particulate 30 is, for example, fluorine resin such as polytetrafluoroethylene, or minute particles such as alkylated silica particles, carbon black, polyvinylidene fluoride, and poly[2-(perfluorooctyl)ethyl acrylate].

In addition, as the water-repellent solid particulate 30, for example, a silicone monolith substance of aerogel or xerogel with polysiloxane structure is applicable. The silicone monolith substance can be obtained by, for example, adopting both bifunctional alkoxysilane and trifunctional alkoxysilane or tetrafunctional alkoxysilanes as starting materials, and copolymerization of such silanes by a sol-gel reaction. An example of such a silicone monolith substance is a silicone monolith substance of aerogel or xerogel obtained from vinyltrimethoxysilane and methylvinyldimethoxysilane. Such materials are described in the report by HAYASE, et al. (Angew. Chem. Int. Ed. Engl. 2013, 52 (41), pp. 10788-10791).

Moreover, a water-repellent and oleophobic solid particulate 30 is also applicable. In the present specification, the term water-repellent and oleophobic means having water-repellent characteristics and oil-repellent characteristics. For example, the solid particulate 30 that indicates a contact angle to water that is equal to or greater than 70 degrees, preferably equal to or greater than 100 degrees, and indicates a contact angle to n- hexadecane that is equal to or greater than 70 degrees, preferably equal to or greater than 100 degrees can be considered as being water-repellent and oleophobic.

An example water-repellent and oleophobic solid particulate 30 is a silicone monolith substance of a material having a polysiloxane structure and a perfluoroalkyl structure, preferably, aerogel or xerogel having a polysiloxane structure and a perfluoroalkyl structure. As for such a material, marshmallow gel that is a flex porous material indicating water-repellent characteristics and oleophobic characteristics and described in the report by HAYASE, et al. (Angew. Chem. Int. Ed. Engl. 2013, 52(41), pp. 10788-10791) is applicable. In addition, as the water-repellent and oleophobic solid particulate 30, for example, particulates of carbon, polyvinylidene fluoride, and the like having a surface modified by perfluoroalkyl group is also applicable.

As for the combination of the wall material precursor liquid 21 and the solid particulate 30, the wall material precursor liquid 21 is preferably hydrophobic and the solid particulate 30 is preferably water-repellent and oleophobic, or the wall material precursor liquid 21 is preferably hydrophilic and the solid particulate 30 is preferably water-repellent or water-repellent and oleophobic.

Next, the encapsulation in the above-described encapsulating step S3 is described.

The encapsulation in the present embodiment, more specifically, the solidification of the wall material precursor liquid 21 can be accomplished by, for example, (a) gelation of the wall material precursor liquid 21, (b) polymerization of the wall material precursor liquid 21, (c) elimination of the solvent contained in the wall material precursor liquid 21, or a combination of two or more of (a) to (c) selected as appropriate.

As illustrated in FIG. 2, the capsule production device 100 may include a solidification facilitating device 140 that accomplishes the facilitation of the solidification. The solidification facilitating device 140 facilitates the solidification of the wall material precursor liquid 21 in the liquid marble 40 moving in the cylindrical body 111 in one direction in the above-described encapsulating step S3. A more specific description is given below.
(a) When the wall material precursor liquid 21 has characteristics to turn in a gel upon being subjected to a temperature change, the solidification facilitating device 140 applies a temperature change to the liquid marble 40 moving in the cylindrical body 111 in one direction. This facilitates the gelation of the wall material precursor liquid 21.

More specifically, the solidification facilitating device 140 cools or heats the liquid marble 40. The cooling can be accomplished by, for example, Peltier element, refrigeration cycle, and the like. The heating can be accomplished by, for example, Peltier element, infrared lamp, and the like.

When, however, the capsule production device 100 is placed under a temperature environment at a room temperature that is, for example, equal to or higher than 15°C and equal to or lower than 25°C, and the gelation of the wall material precursor liquid 21 occurs voluntary due to asymptotic action, to the room temperature, of the wall material precursor liquid 21 heated to a temperature higher than the room temperature in advance or of the wall material precursor liquid 21 cooled to a temperature lower than the room temperature in advance, the solidification facilitating device 140 is unnecessary.

(b) When the wall material precursor liquid 21 contains a monomer, the solidification facilitating device 140 executes a process of facilitating the polymerization of the wall material precursor liquid 21 to the liquid marble 40 that is moving in the cylindrical body 111 in one direction.

More specifically, in the case of photopolymerization that facilitates polymerization by light, the solidification facilitating device 140 emits such light to the liquid marble 40. A structure that causes light emitted from the exterior of the cylindrical body 111 to pass through the cylindrical body 111, and to be incident upon the liquid marble 40 may be employed. The wavelength of light is, for example, 380 nm to 780 nm. Moreover, in the case of heat polymerization that facilitates polymerization by heating, the solidification facilitating device 140 heats the liquid marble 40. A heating temperature is, for example, 30°C to 80°C.

When, however, the polymerization of the wall material precursor liquid 21 that is a monomer occurs voluntary under an environment where the capsule production device 100 is placed, the solidification facilitating device 140 is unnecessary.

Note that, as for the monomer, both a hydrophobic monomer and a hydrophilic monomer are applicable. In the present specification, the hydrophobic monomer refers to a monomer having solubility less than 2 mass% relative to water of 25°C, and the hydrophilic monomer refers to a monomer having solubility equal to or higher than 2 mass% relative to water of 25°C.

Example hydrophobic monomers are: monofunctional acrylate, such as isopropyl acrylate, n-butyl acrylate, isobutyl acrylate, n-amyl acrylate, isoamyl acrylate, n-hexyl acrylate, 2-ethylhexyl acrylate, n-octyl acrylate, decyl acrylate, or dodecyl acrylate; multifunctional acrylate, such as ethylene glycol diacrylate, polyethylene glycol diacrylate, trimethylol propane triacrylate, or pentaerythritol tetraacrylate; monofunctional methacrylate, such as ethyl methacrylate, propyl methacrylate, n-butyl methacrylate, isobutyl methacrylate, n-amyl methacrylate, n-hexyl methacrylate, 2-ethyl hexyl methacrylate, n-octyl methacrylate, or decyl methacrylate; multifunctional methacrylate, such as ethylene glycol dimethacrylate, polyethylene glycol dimethacrylate, trimethylol propane trimethacrylate, or pentaerythritol tetramethacrylate; a styrene-based monomer, such as styrene, α-methyl styrene, vinyl toluene, t-butyl styrene, or chloromethyl styrene; and divinylbenzene. The hydrophobic monomer may be applied alone, or two or more kinds thereof may be applied together. As for the hydrophobic monomer, (meta) acrylate, styrene and divinylbenzene are preferable from the standpoint of the mechanical strength of the wall material 12 of the capsule 10. In the present specification, (meta) acrylate refers to monofunctional or multifunctional acrylate and/or methacrylate.

Example hydrophilic monomers are acrylic acid, methacrylic acid, maleic acid, fumaric acid, vinyl sulfonic acid, styrene sulfonic acid, vinyl alcohol, acrylic amide, and methacryloroxyethylphosphate. The hydrophilic monomer may be applied alone, or two or more kinds thereof may be applied together.

The wall material precursor liquid 21 may also contain constituents, other than a monomer, a polymerization initiator, such as a thermal polymerization initiator or a photopolymerization initiator, a solvent to dissolve or disperse monomers, a surfactant, an UV absorber, a light stabilizer, an oxidation inhibitor, a fire retarder, a plasticizer, wax, and the like.

Example thermal polymerization initiators are: azo compound, such as 2,2'-azobis(4-methoxy-2,4-dimethylvaleronitrile), 2,2'-azobis(2,4-dimethylvaleronitrile), dimethyl-2,2'-azobis(2-methyl propionate), 2,2'-azobis(2-methylbutyronitrile), 1,1'-azobis(cyclohexane-1 -carbonitrile), 2,2'-azobis[N-(2-propenyl)2-methypropioneamide], 1-[(1-cyano-1-methylethyl)azo]formamide, 2,2'-azobis (N-butyl-2-methypropioneamide), or 2,2'-azobis (N-cyclohexyl-2- methypropioneamide); and peroxide, such as t-butyl peroxybenzoate, or 2,5-dimethyl-2,5-di(t-butylperoxy) hexane. Among these examples, azo compound is preferable, and 2,2'-azobis(4-methoxy-2,4-dimethylvaleronitrile) is more preferable. The blending amount of the thermal polymerization initiator is preferably equal to or greater than 0.01 mol% and equal to or less than 5 mol% relative to a monomer.

Example photopolymerization initiators are: acetophenone-based compound like diethoxy acetophenone; benzoin-based compound, such as benzoin, benzoin methyl ether, or benzoin isopropyl ether; acyl-phosphine-oxide-based compound like 2,4,6-trimethyl benzoin diphenyl phosphine oxide; benzophenone-based compound, such as benzophenone, or hydroxy benzophenone; thioxanthone-based compound, such as 2-isopropyl thioxanthone, or 2,4-dimethyl thioxanthone; amino-benzophenone-based compound like 4,4'-diethylamino benzophenone; 10-butyl-2-chloroacridone; 2-ethyl anthraquinone; 9,10-phenanthrenequinone; and camphorquinone. The blending amount of photopolymerization initiator is preferably equal to or greater than 0.01 mol% and equal to or less than 5 mol% relative to a monomer.

The photopolymerization initiator can be applied for a monomer in combination with a photopolymerization promoter as needed in order to make the solidification of the monomer further efficient.

Example photopolymerization promoters are: benzoic-acid-based compound, such as 4-dimethylamino benzoic acid, 4-dimethyl ethyl aminobenzoic acid, 4-dimethylamino benzoic acid n- butoxyethyl, 4-dimethylamino benzoic acid isoamyl, 4-dimethylamino benzoic acid 2-ethylhexyl; and tertiary amine compound, such as triethanolamine, methyl diethanolamine, triisopropanol amine, 4,4'-dimethylamino benzophenone, or 4,4'-diethylamino benzophenone. The blending amount of the photopolymerization promoter is preferably equal to or greater than 0.01 mol% and equal to or less than 5 mol% relative to a monomer.

(c) When the wall material precursor liquid 21 contains a solvent, the solidification facilitating device 140 heats the liquid marble 40 that is moving the cylindrical body 111 in one direction in order to facilitate the elimination of the solvent contained in the wall material precursor liquid 21. When, however, the vaporization of the solvent progresses voluntary under the environment where the capsule production device 100 is placed, the solidification facilitating device 140 is unnecessary.

Note that an example wall material precursor liquid 21 that contains a solvent is a polymer solution in which a polymer is dissolved in an organic solvent.

Example polymers applicable include a polymer derived from the above-described hydrophobic monomer or hydrophilic monomer, preferably poly(meta)acrylate, polystyrene and poly-divinylbenzene. Moreover, polylactic acid, polyglycolic acid, a copolymer of lactic acid and glycolate, polycaprolactone, and the like are also applicable.

Example organic solvents applicable are acetone, methanol, ethanol, dimethyl sulfoxide, dichloroethane, dichloromethane, hexane, toluene, xylene, ethyl acetate, chloroform, diethyl ether, and the like.

The polymer solution may contain constituents, other than a polymer and a solvent, a surfactant, an UV absorber, a light stabilizer, an oxidation inhibitor, a fire retarder, a plasticizer, wax, and the like.

### [Example A]

By utilizing the capsule production device 100 illustrated in FIG. 2, the mononuclear capsule 10 illustrated in FIG. 1A was produced. The inclusion substance 11 is Medium Chain Triglyceride (MCT) that is middle chain fatty acid. The wall material 12 is agarose gel. As for the wall material precursor liquid 21, an agarose aqueous solution with a concentration that is 3 mass% and is heated to a higher temperature than the room temperature in advance was applied. The density of the agarose aqueous solution is greater than the density of MCT.

The amount of the raw material liquid 20 dropped by the dropping device 130 at a time was adjusted so as to be equal to or greater than 8 µL and equal to or less than 10 µL. The dropping device 130 dropped the amount of 0.5 mL of droplets per a minute. The wall material precursor liquid 21 occupied the liquid droplet of the raw material liquid 20 by equal to or greater than 85 volume % and equal to or less than 95 volume %, and the inclusion substance 11 occupied the remaining. As for the solid particulate 30, a fluorine-resin-made substance with a diameter that was substantially 6 ± 2µm was applied.

As the liquid droplets of the raw material liquid 20 rolled in the guide groove 112 together with the fluorine-resin-made solid particulates 30, the liquid droplets of the raw material liquid 20 were dusted with the fluorine-resin-made solid particulates 30, and thus the liquid marbles 40 were formed.

While the liquid marbles 40 were rolling in the guide groove 112 and were moving in one direction, the agarose aqueous solution that is the wall material precursor liquid 21 was cooled. Hence, the agarose aqueous solution turned into a gel. Consequently, the mononuclear capsule 10 that has the wall material 12 formed of the agarose gel was obtained. The capsule 10 were ejected from an end portion of the cylindrical body 111 in sequence.

Subsequently, in order to further solidify the wall material 12, a drying process of repelling moisture in the wall material 12 in a gel shape was executed on each of the capsule 10 ejected from the cylindrical body 111.

In the above case, in order to find an optimized circumferential velocity of the inner surface of the cylindrical body 111, the circumferential velocity of the inner surface of the cylindrical body 111 was changed variously. The circumferential velocity of the inner surface of the cylindrical body 111 being 0.08 m/s is defined as Example A1, the circumferential velocity of the inner surface of the cylindrical body 111 being 0.13 m/s is defined as Example A2, the circumferential velocity of the inner surface of the cylindrical body 111 being 0.19 m/s is defined as Example A3, the circumferential velocity of the inner surface of the cylindrical body 111 being 0.24 m/s is defined as Example A4, the circumferential velocity of the inner surface of the cylindrical body 111 being 0.32 m/s is defined as Example A5, and the circumferential velocity of the inner surface of the cylindrical body 111 being 0.42 m/s is defined as Example A6.

In addition, as for the capsules 10 obtained in Examples A1 to A6, the sphericity that represents the approximation to the true sphere in shape, the wall-thickness uniformity that represents the uniformity of the thickness of the wall material 12, the success rate of the production of the capsule 10, and the inclusion-substance content percentage that is the content percentage of the inclusion substance 11 were measured. The definitions of those evaluation indices are specifically described below.

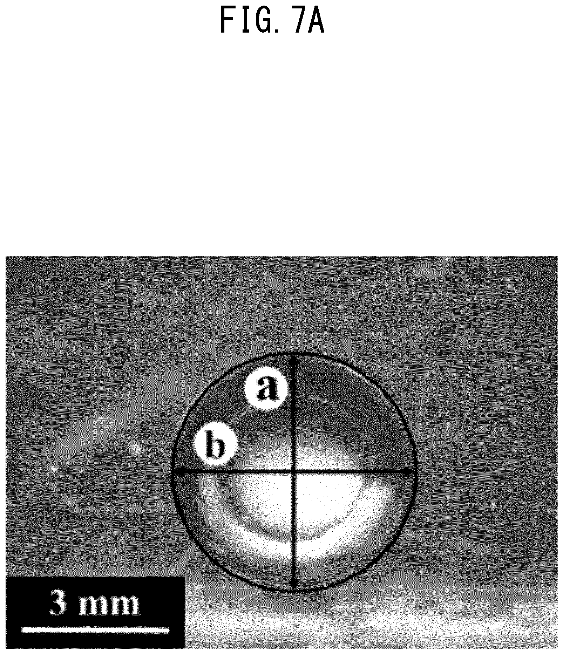

With reference to FIG. 7A, the definition of the sphericity is described. In the micrograph of the capsule 10 after the above-described drying process, a longest diameter a of the capsule 10 in a spherical shape, and a shortest diameter b are measured. The sphericity [%] is defined as b/a × 100.

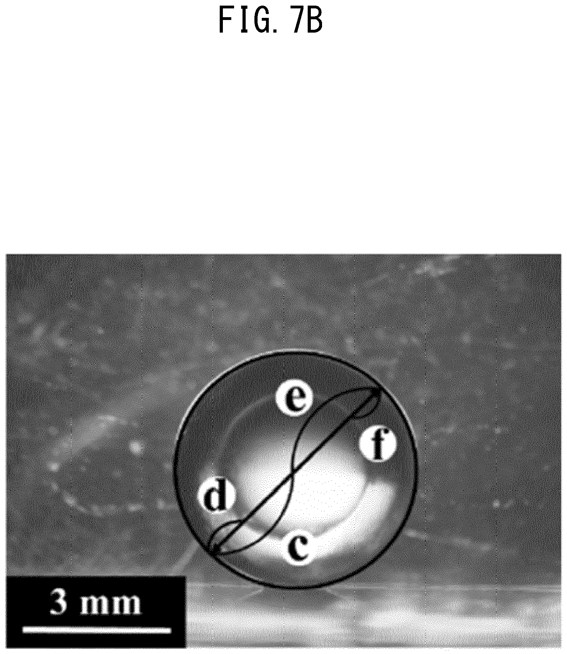

With reference to FIG. 7B, the definition of the wall-thickness uniformity is described. In the micrograph of the capsule 10 after the drying process, a thickness d of the wall materials 12 at the part where the wall material 12 is thickest, a radius c of the position including a line that represents the thickness d, a thickness f of the wall material 12 at a part where the wall material 12 is thinnest, and a radius e of the position including the line that represents the thickness f are measured. The wall-thickness uniformity [%] is defined as (f/e)/(d/c) × 100.

The success rate [%] is defined as (the total number of the products recognizable as the capsules 10)/(the total number of the liquid droplets of the raw material liquid 20 dropped by the dropping device 130) × 100. Note that the success rate was obtained at a stage prior to the drying process and at a stage after the drying process. This is because that there is a possibility such that the wall material 12 is damaged by the above-described drying process, and when the damage of the wall materials 12 is confirmed, the leakage of the inclusion substance 11 may occur, and such a case cannot be recognized as the successful production of the capsule 10.

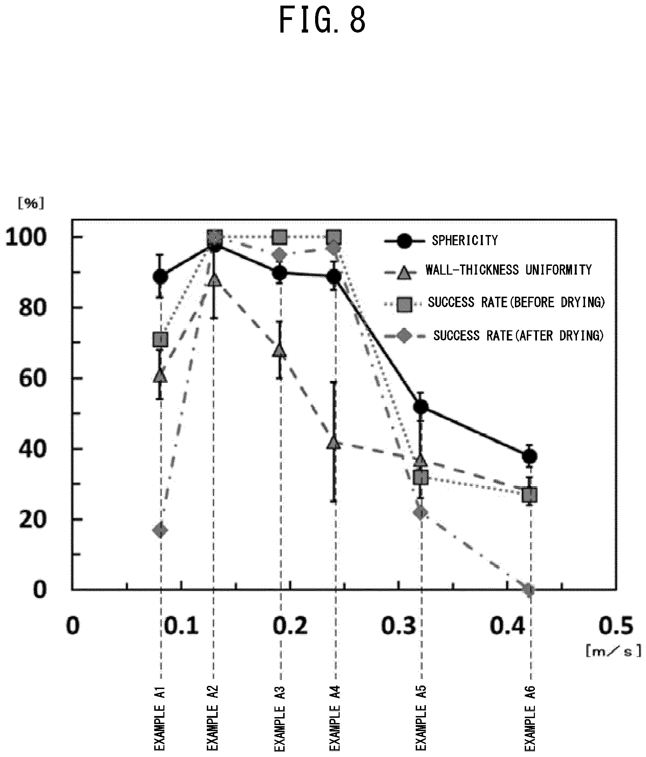

FIG. 8 illustrates the dependence properties of the above-described sphericity, wall-thickness uniformity, and success rate to the circumferential velocity of the inner surface of the cylindrical body 111. The vertical axis in FIG. 8 represents the sphericity [%], the wall-thickness uniformity [%], and the success rate [%], and the horizontal axis represents the circumferential velocity [m/s] of the inner surface of the cylindrical body 111.

As illustrated in FIG. 8, when the circumferential velocity of the inner surface of the cylindrical body 111 is equal to or faster than 0.08 m/s and equal to or slower than 0.24 m/s, the sphericity [%], the wall-thickness uniformity [%], and the success rate [%] relatively become high. In view of such results, when the amount of the raw material liquid 20 to be dropped at a time by the dropping device 130 is equal to or less than 15 µL, the circumferential velocity of the inner surface of the cylindrical body 111 is preferably equal to or faster than 0.08 m/s and equal to or slower than 0.24 m/s. The circumferential velocity of the inner surface of the cylindrical body 111 is more preferably equal to or faster than 0.08 m/s and equal to or slower than 0.19 m/s, most preferably 0.13 m/s.

Note that according to the capsule 10 of Example A2, the sphericity was 98 ± 2 [%], the wall-thickness uniformity was 88 ± 11 [%], and the success rate before the drying process and the success rate after the drying process were 100 [%] and 100 [%]. Moreover, the inclusion-substance content percentage [%] defined by (the mass [g] of the inclusion substance 1 1)/(the mass [g] of the capsule 10 before the drying process) × 100 was 77 [%].

### [Example B]

The capsule 10 was produced under the same conditions as those of Example A except that, as the solid particulates 30, edible material, more specifically, soybean wax powders that contain linoleic acid as primary constituent was applied. The diameter of the soybean wax powder applied as the solid particulate 30 was 64 ± 30 µm. Since the solid particulates 30 were edible, even when the capsule 10 were to be adopted as edible, the solid particulate eliminating step S5 illustrated in FIG. 4 can be omitted. Note that the circumferential velocity of the inner surface of the cylindrical body 111 was set to be 0.13 m/s like the case of Example A2.

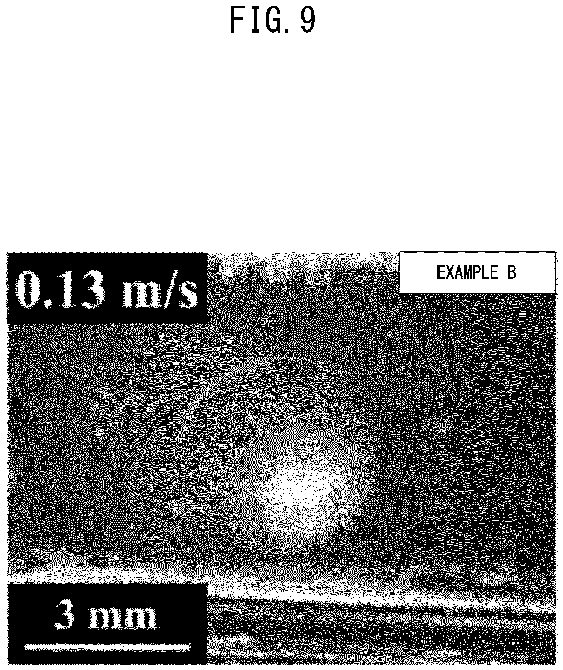

FIG. 9 illustrates a micrograph of the capsule 10 according to Example B. According to the capsule 10 of Example B, the sphericity was 98 ± 2 [%], the wall-thickness uniformity was 91 ± 16 [%], the success rate before the drying process was 100 [%], the success rate after the drying process was 100 [%], and the inclusion-substance content percentage [%] was 71 [%].

### [Example C]

The mononuclear capsule 10 illustrated in FIG. 1A was produced by utilizing the capsule production device 100 illustrated in FIG. 2. The inclusion substance 11 according to the present example was tetradecane that is a heat storage material. The heat storage material is a substance that stores thermal energy, and can release the stored energy when necessary. Moreover, the wall material 12 according to the present example was gelatin. A gelatin aqueous solution with a concentration that was 20 mass% was applied as the wall material precursor liquid 21.

More specifically, the mononuclear capsule 10 was produced under the conditions such that both the discharge rate of tetradecane as the inclusion substance 11 from the dropping device 130 illustrated in FIG. 3, and the discharge rate of the gelatin aqueous solution as the wall material precursor liquid 21 were set to be 0.25 mL/min and the circumferential velocity of the inner surface of the cylindrical body 111 was 0.19 m/s. Other production conditions were the same as those of Example A. Consequently, the mononuclear capsule 10 that contained therein tetradecane as the heat storage material was obtained.

The content percentage of tetradecane in the obtained capsule 10 was 91 [%] with reference to a volume after the drying process. Moreover, according to the capsule 10 of the present example, the sphericity was 98 ± 1 [%], the wall-thickness uniformity was 61 ± 8 [%], and both the success rate before the drying process and the success rate after the drying process were 100 [%].

By the encapsulation that utilizes the heat storage material as the inclusion substance 11 of the capsule 10, in comparison with a case such that the same heat storage material is utilized as a bulk, the specific surface area can be increased. Hence, heat storage and release can be accelerated. Accordingly, the capsule 10 of the present example can contribute to energy saving.

According to conventional scheme of producing a capsule in liquid phase, efficient encapsulation of the heat storage material and increase in the content percentage of the heat storage material in the capsule were difficult. In contrast, according to the present example, the capsule 10 that contains therein the heat storage material can be efficiently obtained in sequence. Moreover, the content percentage of tetradecane in the capsule 10 can be increased to 91 [%].

### [Embodiment 2]

As illustrated in FIG. 2, according to above-described Embodiment 1, the solid particulates 30 are placed on the inner surface of the cylindrical body 111, but the placement of the solid particulate 30 may be omitted. The details thereof are described below.

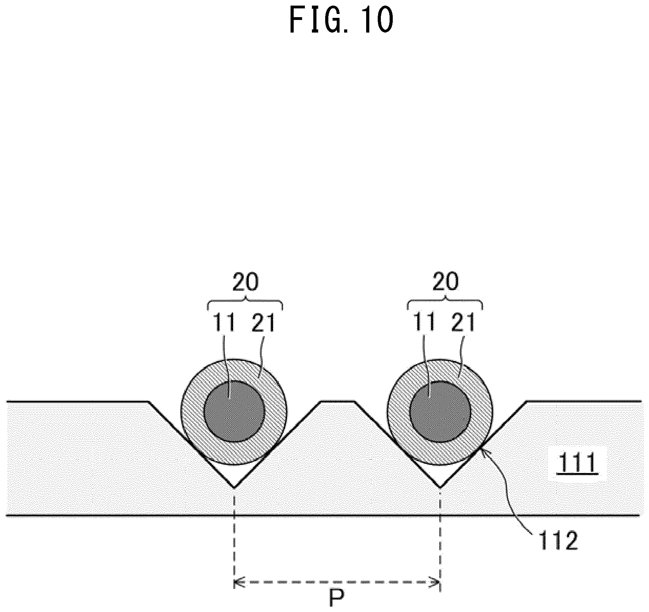

As illustrated in FIG. 10 in an enlarged manner, in the present embodiment, the liquid droplets of the raw material liquid 20 directly contact the inner surface of the guide groove 112 in the interior of the cylindrical body 111. That is, according to the present embodiment, the powder placing step S1 illustrated in FIG. 4 is omitted.

Nevertheless, a clearance is secured between the adjacent liquid droplets of the raw material liquid 20 since a pitch P of the helical guide groove 112 is larger than the diameter of the liquid droplet of the raw material liquid 20. Hence, the unification such that the adjacent liquid droplets of the raw material liquid 20 are unified into a singular droplet can be prevented. Hence, like a case of Embodiment 1, a large number of the capsules can be produced efficiently.

In order to enhance the advantageous effect of suppressing the unification of the liquid droplets of the raw material liquid 20, the pitch P of the guide groove 112 is preferably 1.2 times or more as much as the average diameter of the liquid droplets, more preferably 1.5 times or more, further preferably 2 times or more.

When, for example, the liquid droplets of the raw material liquid 20 is 30 µL, the average diameter of the liquid droplets thereof is substantially 3.8 mm. In this case, the pitch P of the guide groove 112 is preferably equal to or larger than 4.5 mm, more preferably equal to or larger than 5.7 mm, further preferably equal to or larger than 7.6 mm.

Moreover, the liquid droplet of the raw material liquid 20 preferably maintains a substantially spherical shape on the inner surface of the guide groove 112 by own surface tension. In order to do so, the inner surface of the cylindrical body 111 including the inner surface of the guide groove 112 is preferably liquid-repellent to the raw material liquid 20.

When the inner surface of the cylindrical body 111 including the inner surface of the guide groove 112 indicates, to the raw material liquid 20, a contact angle that is equal to or larger than 100 degrees, preferably equal to or larger than 150 degrees, it can be considered that the inner surface of the cylindrical body 111 including the inner surface of the guide groove 112 is liquid-repellent to the raw material liquid 20. More specifically, the cylindrical body 111 itself, or the surface layer portion of the inner surface of the cylindrical body 111 including the surface layer portion of the inner surface of the guide groove 112 may be formed of a material exemplified as the already-described material of the solid particulate 30. This gives the liquid-repellent characteristics to the cylindrical body 111.

### [Embodiment 3]

FIG. 2 illustrates the cylindrical body 111 as an example tubular body that surrounds the virtual center line VL. The cylindrical body 111 has a straight shape such that the distance between the inner surface and the virtual center line VL is constant in the lengthwise direction of the virtual center line VL, that is, the generatrix is in parallel with the virtual center line VL. However, the straight shape is not always requisite for the tubular body. In the present specification, a concept "tubular body" that surrounds the virtual center line VL also includes a shape that has a portion where the distance between the inner surface and the virtual center line VL changes in the lengthwise direction of the virtual center line VL, for example, a tapered shape having the generatrix inclined relative to the virtual center line VL.

Moreover, FIG. 2 illustrates the helical guide groove 112 as an example, but the guide groove where the liquid droplets rolls may circle. In the following, an Embodiment that utilizes a tapered cylindrical body having a circling guide groove in the inner surface is described as another example tubular body.

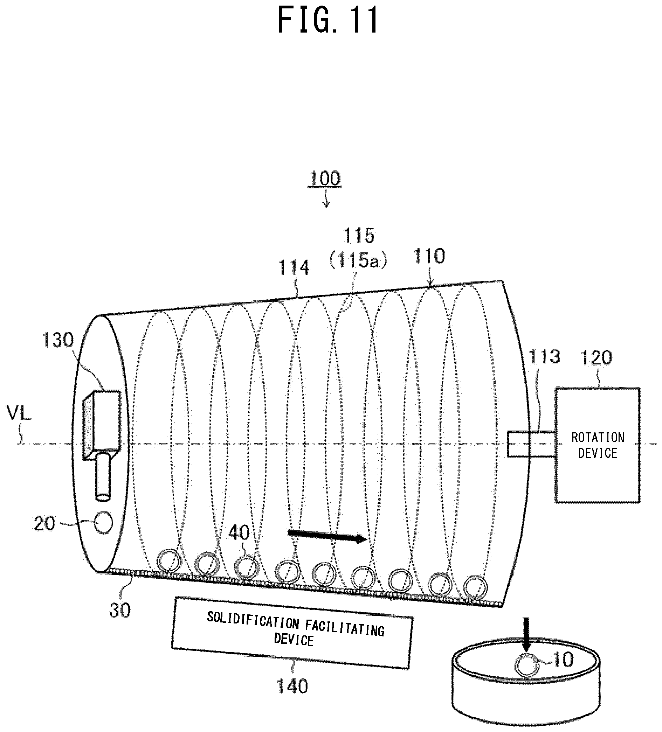

As illustrated in FIG. 11, a cylindrical body 114 of the present embodiment has a spreading shape of which an inner diameter gradually increases from an end portion at a side where the dropping device 130 is placed toward an end portion at a side where the capsule 10 is ejected relative to the parallel direction to the virtual center line VL. Moreover, a guide groove 115 according to the present embodiment includes a plurality of circling grooves 115a arranged side by side in the lengthwise direction parallel to the virtual center line VL.

According to the present embodiment, also, each of the liquid droplets of the raw material liquid 20 also rolls in the guide groove 115 in the form of the liquid marble 40. Since the generatrix of the cylindrical body 114 is inclined downwardly as becoming apart from the dropping device 130, each rolling liquid marble 40 slides in the circumferential direction of the cylindrical body 115, and moves downwardly along the generatrix by own weight. Moreover, during such a movement, the solidification of the wall material precursor liquid 21 in each liquid marble 40 progresses. Other structures and actions are similar to those of Embodiment 1.

Embodiments and Examples are described above. Modified Examples (1) to (5) to be described below are also adoptable.
(1) FIG. 3 illustrates an example liquid droplet of the raw material liquid 20 that has the mononuclear inclusion substance 11 covered by the wall material precursor liquid 21. This liquid droplet is suitable for the production of the capsule 10 illustrated in FIG. 1A. For example, the dropping nozzle portion that drops the raw material liquid 20 in the dropping device 130 may include multiple pipes that are triple pipes or more. This enables a formation of a liquid droplet of the raw material liquid 20 in a form that has the multinuclear inclusion substance 11 covered by the wall material precursor liquid 21. Such a liquid droplet is suitable for the production of the capsule 10 illustrated in FIG. 1B. Since the encapsulation of the raw material liquid 20 progresses while the liquid droplet of the raw material liquid 20 rolls, when the multinuclear capsule 10 illustrated in FIG. 1B is produced, displacement of the inclusion substance 11 that becomes a nucleus is not likely to occur.
   Moreover, by applying the raw material liquid 20 in a form such that the inclusion substance 11 is dispersed in the wall material precursor liquid 21 in a molecular level, or the raw material liquid 20 in a form such that the inclusion substance 11 is dissolved in the wall material precursor liquid 21, the capsule 10 illustrated in FIG. 1C can be produced. Since the encapsulation of the raw material liquid 20 progresses while the liquid droplets of the raw material liquid 20 roll, when the capsule 10 illustrated in FIG. 1C is to be produced, the internal composition can be made uniform.
(2) Moreover, FIG. 3 illustrates an example structure such that the inclusion substance 11 and the wall material precursor liquid 21 are dropped together, but the inclusion substance 11 and the wall material precursor liquid 21 may be dropped separately. Encapsulation may be carried out while the inclusion substance 11 and the wall material precursor liquid 21 that are dropped separately are rolling in the guide groove 112. In this case, the concept of "encapsulation" includes the covering of the inclusion substance 11 by the wall material precursor liquid 21 and the solidification of the wall material precursor liquid 21 that is covering the inclusion substance 11. Note that when the inclusion substance 11 and the wall material precursor liquid 21 are dropped separately, the already-described powder placing step S1 may be omitted.
(3) FIG. 2 and FIG. 11 illustrate, an example rotating tube 110 having the virtual center line VL placed horizontally. The virtual center line VL may have a slight inclination angle θ, more specifically, an inclination angle θ that is, for example, equal to or smaller than 60 degrees, preferably equal to or smaller than 30 degrees relative to the horizontal plane. In the present specification, the meaning of the "attitude falling sideways" also includes a case such that the virtual center line VL has such a slight inclination angle θ. For example, in the structure illustrated in FIG. 2, by causing the virtual center line VL to have an inclination angle θ, the cylindrical body 111 may be inclined downwardly from the end portion at a side where a liquid droplet is dropped toward the end portion at a side where the capsule 10 is ejected.

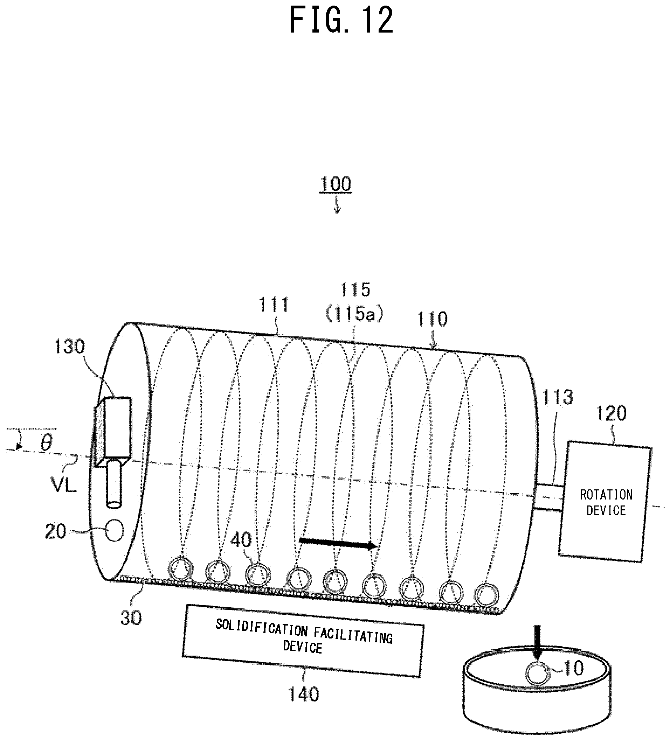

   FIG. 12 illustrates the cylindrical body 111 having the virtual center line VL with such an inclination angle θ. Each liquid marble 40 rolling on the inner surface of the cylindrical body 111 slides in the circumferential direction of the cylindrical body 111, and moves downwardly by own weight along the generatrix parallel to the virtual center line VL. According to this structure, like the case of Embodiment 3, the guide groove 115 may be configured by the plurality of circling grooves 115a arranged side by side along the lengthwise direction parallel to the virtual center line VL.
(4) FIG. 11 and FIG. 12 illustrate example structures such that the circling guide groove 115 is provided for the cylindrical bodies 114 and 111 each having a generatrix inclined relative to the horizontal plane. The helical guide groove 112 may be provided for the cylindrical bodies 114 and 111 each having a generatrix inclined relative to the horizontal plane. Moreover, FIG. 2 illustrates an example structure such that the helical guide groove 112 is provided for the cylindrical body 111 having the generatrix placed horizontally, but the similar advantageous effects can be accomplished when the circling guide groove 115 is provided for the cylindrical body 111 having the generatrix placed horizontally.
(5) FIG. 2 and FIG. 11 illustrate, as an example tubular body that surrounds the virtual center line VL, the cylindrical bodies 111 and 114 with a circular cross section that is perpendicular to the virtual center line VL. In the present specification, "circular" is a concept including not only a true circle but also an ellipse. However, the circular cross section of the "tubular body" surrounding the virtual center line VL is not requisite.

Specific examples of the above-described inclusion substance 11 are described below.

The inclusion substance 11 can be selected in accordance with the application of the capsule 10. As for the inclusion substance 11, the oil-soluble substance or the water-soluble substance are both applicable. The inclusion substance 11 may be applied alone, or two or more kinds may be applied together.

Example oil-soluble inclusion substances 11 are oil-soluble agricultural chemical, adhesive, ink, heat storage material, pharmaceutical product, and the like. More specifically, for example, a cold medium like tetradecane, liposoluble vitamin groups like α-tocopherol, and an anticancer drug like doxorubicin are applicable as the oil-soluble inclusion substance 11.

Example water-soluble inclusion substances 11 are water-soluble agricultural chemical, adhesive, ink, heat storage material, pharmaceutical product, and the like. More specifically, a solvent like water, and protein substance like bovine serum albumin are applicable as the water-soluble inclusion substance 11.

The inclusion substance 11 may be volatile or nonvolatile. Moreover, the inclusion substance 11 may be applied in a state dissolved in a solvent. The solvent can be selected in accordance with the applied inclusion substance 11. When the inclusion substance 11 is oil-soluble, for example, organic solvents, such as acetone, methanol, ethanol, dimethyl sulfoxide, dichloroethane, dichloromethane, hexane, xylene, ethyl acetate, chloroform, and diethyl ether, are applicable. When the inclusion substance 11 is water-soluble, for example, a solvent like water is applicable.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2021-98149 filed on June 11, 2021, the entire disclosure of which is incorporated by reference herein.

### Reference Signs List

- 10: Capsule
- 11: Inclusion substance
- 12: Wall material
- 13: Composite matrix
- 20: Raw material liquid
- 21: Wall material precursor liquid
- 30: Solid particulate
- 40: Liquid marble
- 100: Capsule production device
- 110: Rotating tube
- 111: Cylindrical body (tubular body)
- 112: Guide groove
- 113: Rotation shaft
- 114: Cylindrical body (tubular body)
- 115: Guide groove
- 115a: Circling groove
- 120: Rotation device
- 130: Dropping device
- 131: Inner pipe
- 132: Outer pipe
- 140: Solidification facilitating device
- VL: Virtual center line

## Claims

1. A capsule production method to be performed by utilizing a tubular body having a shape of tube surrounding a virtual center line, the tubular body including a guide groove that is helical or circles about the virtual center line, the guide groove being provided in an inner surface of the tubular body, the method comprising:
a dropping step of dropping, on the inner surface of the tubular body, an inclusion substance and a wall material precursor liquid that is a precursor of a wall material covering the inclusion substance;
an encapsulating step of progressing an encapsulation of the inclusion substance and the wall material precursor liquid by a liquid droplet dropped in the dropping step rolling in the guide groove of the tubular body, the tubular body rotating around the virtual center line; and
a capsule ejecting step of ejecting, from the tubular body, a capsule obtained by a progress of the encapsulation.

2. The capsule production method according to claim 1, further comprising:
a powder placing step, prior to the dropping step, of placing powders of solid particulates in the guide groove of the tubular body in advance, the solid particulates being each smaller than the liquid droplet, wherein
in the encapsulating step, a liquid marble is formed by the liquid droplet rolling together with the solid particulates in the guide groove of the tubular body rotating, and the liquid droplet rolls in the guide groove in a form of the liquid marble, the liquid marble being the liquid droplet having a surface that is dusted with the solid particulates.

3. The capsule production method according to claim 1, wherein a surface of the guide groove of the tubular body is liquid-repellent to the liquid droplet.

4. The capsule production method according to claim 1 or 2, wherein
an amount of the liquid droplet to be dropped at a time in the dropping step is equal to or less than 15 µL, and
a circumferential velocity of the inner surface of the tubular body in the dropping step and in the encapsulating step is equal to or faster than 0.08 m/s and equal to or slower than 0.24 m/s.

5. The capsule production method according to any one of claims 1 to 4, wherein
the wall material precursor liquid has a characteristic of gelation, and
the encapsulation progresses by the gelation of the wall material precursor liquid.

6. The capsule production method according to any one of claims 1 to 4, wherein
the wall material precursor liquid contains a monomer, and
the encapsulation progresses by polymerization of the monomer.

7. The capsule production method according to any one of claims 1 to 4, wherein
the wall material precursor liquid contains a solvent, and
the encapsulation progresses by elimination of the solvent.

8. A capsule production device comprising:
a tubular body having a shape of tube surrounding a virtual center line, the tubular body including a guide groove that is helical or circles about the virtual center line, the guide groove being provided in an inner surface of the tubular body;
a rotation device to cause the tubular body to rotate around the virtual center line; and
a dropping device to drop, on the inner surface of the tubular body, an inclusion substance and a wall material precursor liquid that is a precursor of a wall material covering the inclusion substance, wherein
an encapsulation of the inclusion substance and the wall material precursor liquid progresses by a liquid droplet dropped by the dropping device rolling in the guide groove of the tubular body being rotated by the rotation device.

9. The capsule production device according to claim 8, wherein an amount of the liquid droplet to be dropped at a time by the dropping device is equal to or less than 15 IlL.

10. The capsule production device according to claim 8 or 9, further comprising:
a solidification facilitating device to facilitate solidification of the wall material precursor liquid in the liquid droplet that is rolling in the guide groove of the tubular body.
